# EUROPÄISCHE PATENTANMELDUNG

(11) **EP 0 711 566 A1**
(43) Veröffentlichungstag der Anmeldung: **15.05.1996**
(21) Anmeldenummer: 94117949.1
(22) Anmeldetag: 14.11.1994
(51) Int. Cl.: A61L 2/00, A61C 19/00

(54) **Verfahren und Einrichtung zur Reinigung und Entkeimung eines zahnärztlichen Gerätekopfes**

(71) Anmelder: SIEMENS AKTIENGESELLSCHAFT, D-80333 München (DE)
(72) Erfinder: Franetzki, Manfred Dr.-Ing, Dipl.-Phys., D-64625 Bensheim (DE)

(57) **Zusammenfassung**

Um auf eine praktikable und ökonomisch vertretbare Weise eine gezielte Reinigung und Entkeimung aller, einer Kontamination ausgesetzter Teile eines zahnärztlichen Gerätekopfes erzielen zu können, wird folgendes vorgeschlagen:
Zumindest die Instrumente mit den daran angeschlossenen Schläuchen, vorzugsweise auch ein vorhandenes Bedienfeld, sowie Handhabemittel zur Verstellung des Geräteskopfes, werden in betriebsmäßigem Zustand in eine verschließbare Desinfektorkammer gebracht, in der sie gereinigt und außen und innen entkeimt werden. Die Teile werden zunächst von außen durch Abstrahlen mit einem Reinigungsmedium gereinigt. Anschließend werden die Außenflächen sowie die Zufuhrleitungen in den Schläuchen und die Kanäle in den Instrumenten innen mit einem Desinfektionsmedium entkeimt.

## Beschreibung

Die Erfindung bezieht sich auf ein Verfahren zur Reinigung und Entkeimung eines zahnärztlichen Gerätekopfes, der Ablagehalterungen für schlauchgebundene Instrumente umfaßt, der ein Bedienfeld enthält und der mit einer Handhabe ausgestattet ist, mittels der er in unterschiedliche Behandlungsstellungen positionierbar ist. Die Erfindung bezieht sich außerdem auf eine Einrichtung zur Durchführung eines solchen Verfahrens.

Bei zahnärztlichen Behandlungen besteht die Gefahr der Keimübertragung im wesentlichen auf drei Wegen:
Bei Präparationen im Patientenmund können durch den beim Kühlen der Präparationsstelle verwendeten Spray mit Keimen kontaminierte Partikel aus dem Patientenmund herausgeschleudert werden wodurch das Behandlungspersonal direkt mit Keimen kontaminiert wird.Gegen eine Keimübertragung auf diesem Wege kann sich das Personal durch geeignete Maßnahmen, wie Brillen und Masken, schützen.

Keime, die über das Wasserleitungsnetz oder über die Behandlungsinstrumente in die Versorgungskanäle gelangen und sich dort vermehren, können durch biozide Zuschlagstoffe in den Versorgungsmedien abgetötet werden. Ein hierfür geeignetes Verfahren ist beispielsweise in dem europäischen Patent EP-0 111 249 (GR 82 P 3834 E) beschrieben.

Ein Rücksaugen der Keime in die Medienkanäle der Instrumente bzw. Versorgungsleitungen kann zwar durch an geeigneter Stelle angeordnete Ventile weitgehend verhindert und eine Rekontamination in den Instrumenten durch eine Sterilisation der Instrumente vermieden werden. Zum Sterilisieren der Instrumente müssen diese jedoch in der Regel zunächst von den Versorgungsschläuchen abgekoppelt und danach getrennt in einem Sterilisator oder Desinfektor behandelt werden.

Ein Hauptproblem stellt die Entkeimung derjenigen Teile eines zahnärztlichen Gerätekopfes dar, die zwangsläufig vom Bedienpersonal, gegebenenfalls auch vom Patienten, berührt werden und damit besonders stark einer Verkeimung ausgesetzt sind, die aber nicht in den heute üblichen Desinfektions- bzw. Sterilisationsgeräten eingegeben werden könnnen. Hierunter fallen insbesondere Griffe, Bedienfelder oder sonstige Gehäuseteile, die heute in der Regel lediglich mit einem Desinfektionsspray behandelt werden. Dies gilt im Prinzip auch für Instrumentenhalterungen bzw. Instrumentenablagen.

In der DE-C2-3 440 078 ist zwar eine Dental-Enheit mit einem sterilisierbaren Geräteteil beschrieben, bei der der Gerätekopf von außen per UV-Licht oder mit Hilfe eines trockenen Gases sterilisiert werden kann, indem der Gerätekopf mit den Instrumenten in einen unterhalb der Raumdecke befindlichen Schacht gefahren wird.Der Schacht kann im eingefahrenen Zustand durch einen bodenseitigen Deckel verschlossen werden. Eine anschließende Behandlung beschränkt sich ausschließlich auf eine Trocken-Sterilisation der äußeren Teile; es erfolgt somit keine Reinigung und auch keine Entkeimung der inneren Teile.

Das Ziel einer lückenlosen Entkeimung aller mit Keimen in Verbindung kommender Teile ist somit auch mit dieser Einrichtung nicht erreichbar und wäre mit den zuvor beschriebenen Methoden ökonomisch nicht vertretbar, was in der Praxis zur Folge hat, daß die konsequente Entkeimung in der Regel unterbleibt.

Der im Anspruch 1 angegebenen Erfindung liegt die Aufgabe zugrunde, ein Verfahren zur Reinigung und Entkeimung anzugeben, mit dem auf praktikable und ökonomisch vertretbare Weise eine gezielte Reinigung und Entkeimung sowohl der Medienkanäle als auch sämtlicher anderer, insbesondere äußerer Teile, durchgeführt werden kann.

Gemäß der Erfindung werden sämtliche direkt oder indirekt mit Keimen in Berührung kommenden Teile in situ, d.h. ohne vorherige Demontage der Teile, also im betriebsfähigen Zustand, automatisch gereinigt und anschließend entkeimt. Die Reinigung erfolgt, indem die äußeren Flächen mit Hilfe eines Reinigungsmediums unter Druck abgestrahlt werden. Dies kann vorteilhafterweise mit einem Wasserstrahl oder Dampfstrahl oder im Ultraschallbad erfolgen; denkbar und im Rahmen der Erfindung ist es auch, andere geeignete Reinigungsmittel zu verwenden. Die Zufuhr der Reinigungsmedien erfolgt vorteilhafterweise über ein Düsensysem mit einer Vielzahl von Düsen, die in der verschließbaren Desinfektorkammer angeordnet sind. Die Entkeimung der Zufuhrleitungen und Kanäle in den Instrumenten kann nach der an sich bekannten, eingangs beschriebenen Methode erfolgen.

Die Entkeimung der Außenflächen kann mit geeigneten Mitteln erfolgen, z.B. durch Besprühen mit bioziden Chemikalien, mit H₂0₂ oder mit erhitztem Wasserdampf oder auch durch Beschwallen mit ozoniertem Wasser. Auch ein Beströmen mit Ozongas, Heißluft oder anderen bioziden Gasen oder eine Bestrahlung mit UV-Licht, Mikrowellen oder Beschallen mit Ultraschall ist denkbar.

Gemäß einer vorteilhaften Ausführung werden nicht nur schlauchgebundene Instrumente, Bedienfeld und Handhabe dem Reinigungs- und Entkeimungsverfahren unterworfen, sondern dabei gleichzeitig auch noch weitere, am Gerätekopf angebrachte Teile, insbesondere ein Instrumentenablagetablett, gegebenenfalls einschließlich der daraufliegenden Instrumente, und/oder eine Speischale, mitbehandelt.

Nachfolgend werden mehrere Ausführungsbeispiele der Erfindung beschrieben.

Es zeigen:
Figuren 1 bis 5 ein erstes Ausführungsbeispiel einer erfindungsgemäßen Einrichtung,
Figuren 6 und 7 ein zweites Ausführungsbeispiel,
Figuren 8 bis 10 ein drittes Ausführungsbeispiel,
Figuren 11, 12 und 13 drei weitere Ausführungsbeispiele.

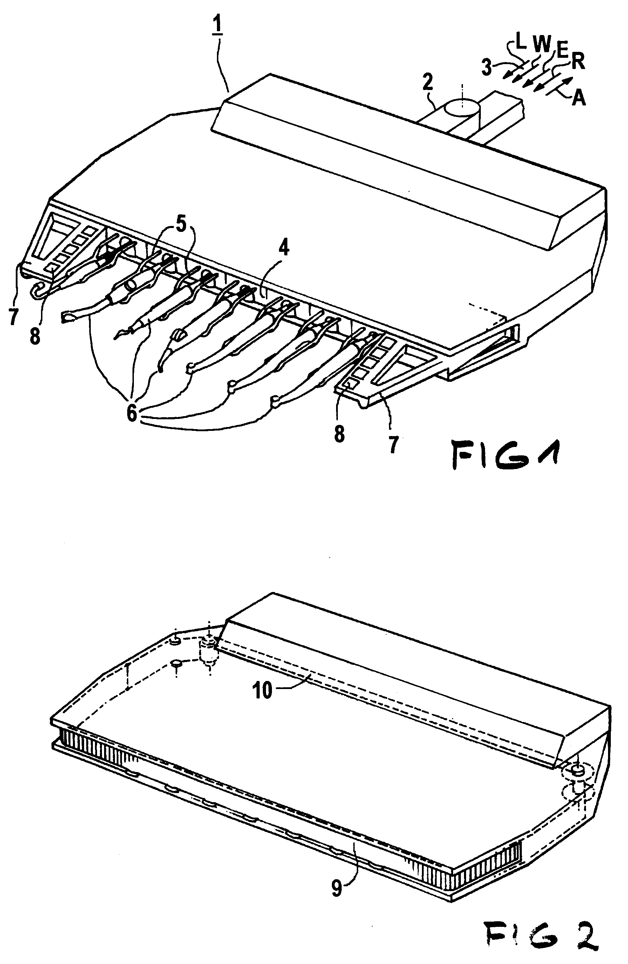

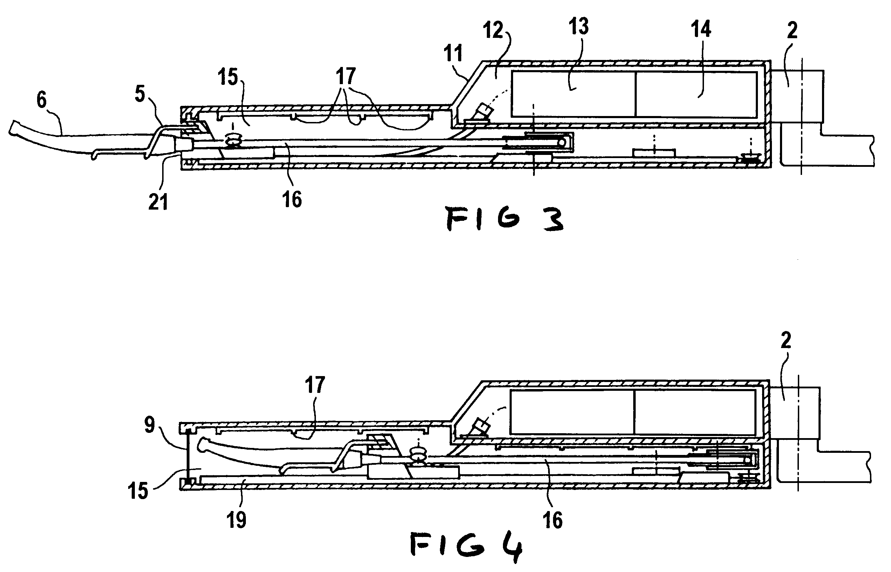

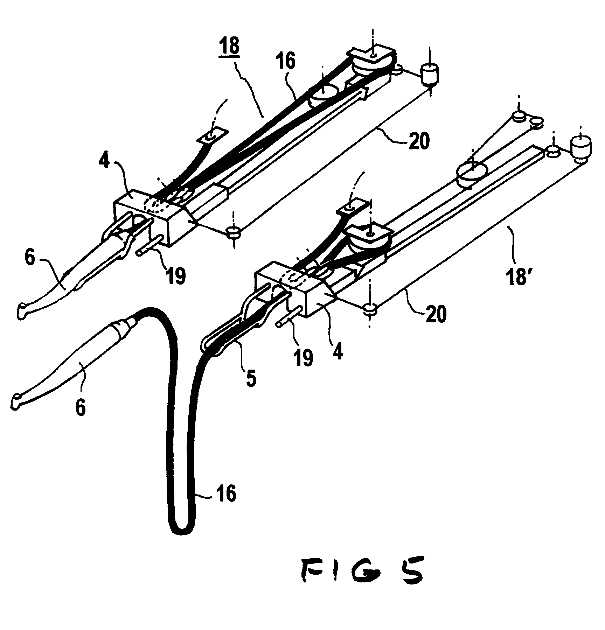

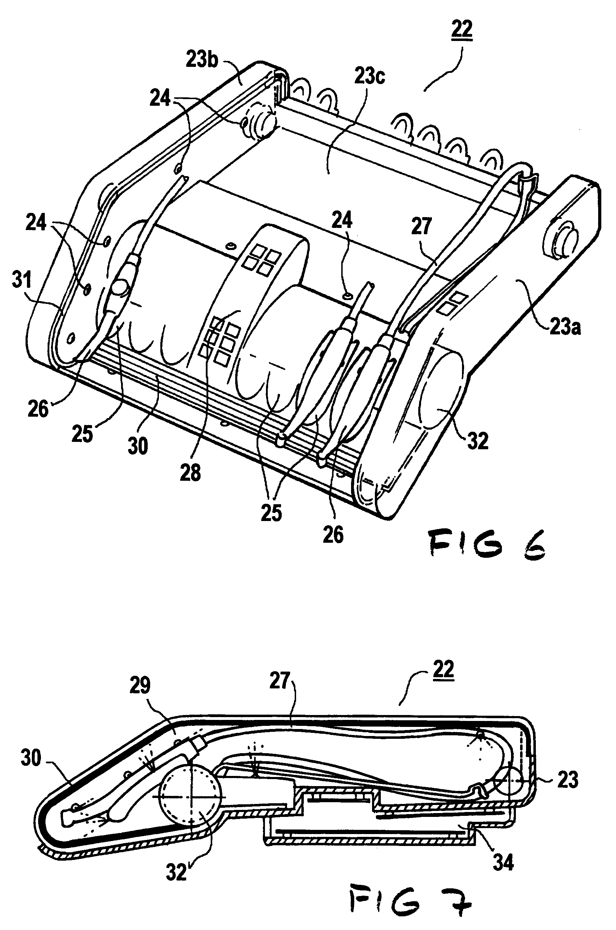

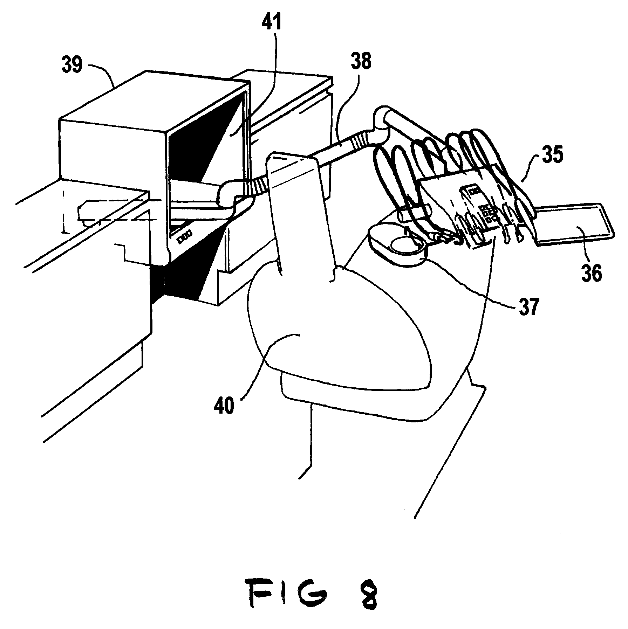

Die Figur 1 zeigt in einer schaubildlichen Darstellung einen zahnärztlichen Gerätekopf 1, der in bekannter Weise mittels eines Tragarmes 2 an einem (nicht gezeigten) Bodenständer oder auch an einem Wand- oder Deckenadapter so gehaltert ist, daß er in verschiedene Behandlungspositionen bringbar ist. Im Tragarm 2 verlaufen in bekannter Weise durch Pfeile 3 angedeutete Zufuhrleitungen, und zwar einerseits für Luft (L), Wasser (W) und elektrische Energie (E) und andererseits für ein Reinigungsmedium (R) sowie eine Abflußleitung für Abwasser (A). An der Bedienseite befindet sich eine Köcherleiste 4 mit einer Vielzahl von Ablagevorrichtungen 5 für diverse schlauchgebundene Instrumente 6. Beidseitig der Instrumentenanordnung befinden sich Griffe 7, die ein integriertes Bedienfeld 8 enthalten. Wie nachfolgend noch näher erläutert, kann die Köcherleiste 4 mit den Instrumenten in das Innere des Geräteskopfes 1 eingezogen werden. Desgleichen können die beiden Griffe mit dem integrierten Bedienfeld nach innen geschwenkt werden. Die verbleibenden Öffnungen werden in dieser Nichtgebrauchsstellung von einer Jalousie 9 abgedeckt, die, wie in Figur 2 angedeutet, ein motorisch angetriebenes Band mit Zugseilen sein kann, welches durch einen Seilzug 10 bewegt wird und in der Gebrauchsstellung (Fig. 1) eine entsprechende schlitzförmige Öffnung (21 in Fig. 3) für den Austritt der genannten Köcherleiste und der Griffe, freigibt und in der in Figur 2 dargestellten Nichtgebrauchsstellung diese Öffnung dicht verschließt.

Die Figuren 3 und 4 zeigen den Gerätekopf in den beiden genannten Stellungen jeweils im Querschnitt. Aus der Darstellung ergibt sich, daß das Gerätekopfgehäuse 11 einen oberen Stauraum 12 aufweist, in dem eine mit 13 bezeichnete Elektronik sowie eine mit 14 bezeichnete Mechanik (Ventile, etc.) untergebracht sind. Nachdem diese Teile für die Erfindung nicht wesentlich sind, sei hier nur erwähnt, daß die Mechanik 14 die für das nachfolgend noch näher geschilderte Verfahren notwendigen Steuerventile enthält, mit denen einerseits der Zufluß der Medien zu den Instrumenten und andererseits die Zufuhr des Reinigungsmediums sowie Abfuhr des Reinigungswassers gesteuert werden kann.

Mit 15 ist ein unterer Stauraum bezeichnet, der in sich geschlossen und frei von elektrischen und elektronischen Bauteilen ist und der lediglich einen Zulauf für ein Reinigungsmedium sowie einen Abfluß für Abwasser enthält. Dieser untere Stauraum 15 bildet bei diesem Ausführungsbeispiel eine Desinfektorkammer, in der bei eingezogenen Instrumenten 6 und Versorgungsschläuchen 16 sowie bei eingeklappten Griffen 7 diese Teile einer automatischen Reinigung und Desinfektion unterzogen werden. Hierzu befindet sich im vorderen Teil der Desinfektorkammer 15 eine Düsenanordnung 17, die mit der Zuleitung für Reinigungsmedium (R) verbunden sind.

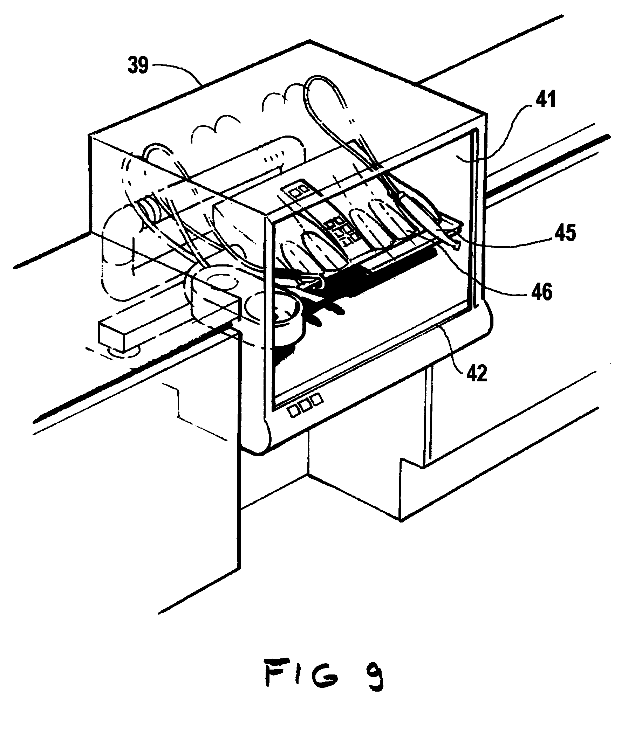

Die Figur 5 zeigt in schematischer Darstellung den Aufbau eines allgemein mit 18 bezeichneten Schlauchzuges, und zwar zum einen bei abgelegtem Instrument 6, zum anderen bei aus der Ablage entnommenem Instrument (18'). Nachdem die Schlauchrückholvorrichtung des Schlauchzuges Stand der Technik ist, braucht diese nicht näher erläutert zu werden. Bezüglich der Verfahrbarkeit der Köcherleiste 4 sei angemerkt, daß diese auf in der Desinfektorkammer 15 befestigten Führungsstangen 19 verfahrbar gelagert sind. Das Ausfahren der Instrumente bzw. der Köcherleiste mit Instrumenten sowie das Einziehen kann vorteilhafterweise über einen motorisch angetriebenen Seilzug 20 erfolgen.

Die Köcherleiste 4 kann einteilig ausgebildet sein, also so, daß alle Instrumente 6 gleichzeitig ein- und ausgefahren werden; ebenso kann die Köcherleiste auch mehrteilig ausgebildet sein, d.h. für jedes Instrument kann eine Köcherleiste vorgesehen sein, die dann getrennt voneinander ein- und ausfahrbar sind.

Der Reinigungs- und Entkeimungsvorgang läuft wie folgt ab:
Nachdem die Instrumente und die beiden Griffe mit Bedienfeld in die Desinfektorkammer 15 eingefahren sind, wird die verbleibende Öffnung 21 mittels der Jalousie 9 abgedeckt. Danach wird über das Düsensystem 17 Reinigungsmedium in Form von unter Druck stehendem Wasser oder Dampf eingeleitet. Die Anordnung der Düsen ist dabei so getroffen, daß einerseits die Instrumente mit den daran angeschlossenen Schläuchen und andererseits die beiden Griffe mit Bedienfeld zunächst außen abgestrahlt werden. Nach Abpumpen des Reinigungswassers werden anschließend die Außenflächen mit einem Desinfektionsmedium beaufschlagt. Gleichzeitig werden die Zufuhrleitungen in den Schläuchen sowie die Kanäle der Instrumente von innen mit einem Desinfektionsmedium durchströmt. Hierzu wird die Zufuhr von Spraywasser zu den Instrumenten im Ventilblock 14 unterbrochen und stattdessen Desinfektionsmittel zu den Instrumenten geleitet. Die in der Desinfektorkammer nach Abpumpen des Reingungswassers vorhandene Restfeuchtigkeit kann durch Einblasen von warmer Luft und/oder durch Absaugen der Luft entfernt werden.

Zur Verstärkung der Desinfektionswirkung kann dem Wasser Ozon beigemischt werden. Ebenso kann alternativ oder ergänzend die Reinigungswirkung durch Ultraschall verstärkt werden, indem an geeigneter Stelle im Stauraum 15 ein Ultraschallgenerator plaziert ist und die Instrumente in einem Flüssigkeitsbad liegen.

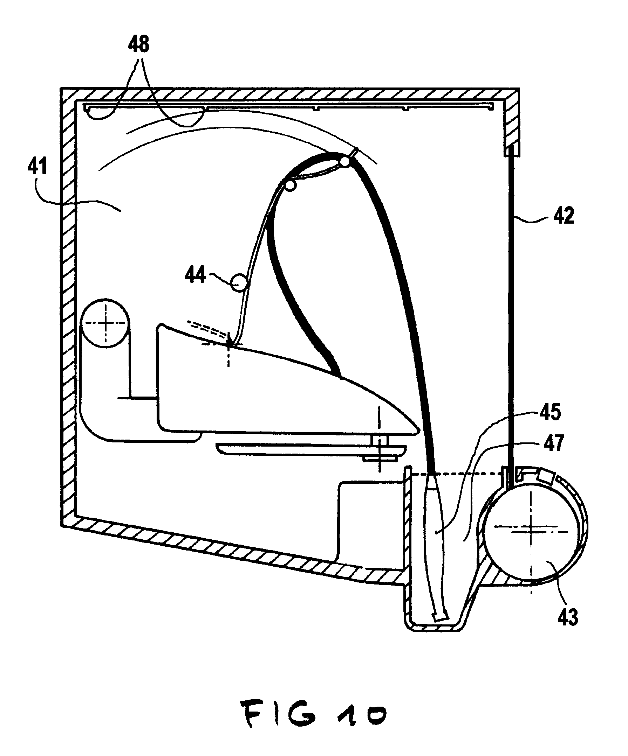

Bei dem anhand der Figuren 6 und 7 beschriebenen zweiten Ausführungsbeispiel läuft das Verfahren zur Reinigung und Desinfektion in gleicher Weise ab. Die Unterschiede zu dem vorangegangenen Ausführungsbeispiel liegen im konstruktiven Aufbau des hier anders gestalteten Gerätekopfes.

Der in den Figuren 6 und 7 gezeigte Gerätekopf 22 enthält ein wannenartig ausgebildetes Gehäuse 23, in dessen beiden Seitenwangen 23a, 23b und Boden 23c Düsen 24 für die Zufuhr von Reinigungsmedium angeordnet sind. Der Gerätekopf enthält ferner Ablagehalterungen 25 für Instrumente 26 und deren Schläuche 27, die nach Art einer Peitsche entnommen und abgelegt werden. Mit 28 ist ein im Träger der Ablagehalterung 25 integriertes Bedienfeld bezeichnet. Die Desinfektorkammer 29 wird hier gebildet einerseits durch das Gehäuse 23 und andererseits durch eine im abgelegten Zustand der Instrumente diese überdeckende und die Wanne abschließende Jalousie 30. Die Jalousie 30 ist in seitlichen Führungen 31 geführt und kann über eine Trommel 32 motorisch ein- und ausgefahren werden. Wie im Schnittbild nach Figur 7 angedeute, sind die elektronischen und elektrischen Bauteile unterhalb des Gehäuses 23 in einem separaten Stauraum 34 untergebracht.

Die Figuren 8, 9 und 10 zeigen eine weitere Ausführungsform eines Gerätekopfes mit einer sog. Peitschenhalterung für die Instrumente, wie zuvor erläutert. Im Gegensatz zu der bereits beschriebenen Ausführungsform enthält dieser Gerätekopf 35 noch zwei zusätzliche Elemente, nämlich ein Ablagetablett (Tray) 36 und eine Speischale 37. Des weiteren wird der Gerätekopf 35 von einem mehrgliedrigen Tragarm 38 getragen, dessen freies Ende im Innern eines kastenartigen Einbauschrankes 39 seitlich neben einem oder am Fußende eines Patientenstuhles 40 gehaltert ist. Der Innenraum 41 bildet hier wiederum eine Desinfektorkammer, in die nicht nur der Gerätekopf samt Instrumententray 36 und Speischale 37, sondern auch der gesamte Tragarm 38 eingefahren werden kann. Zur Abdeckung der Frontseite ist eine Jalousie 42 vorgesehen, die von einer unterhalb der Einbringöffnung befindlichen Trommel 43 aus abgewickelt werden kann. Der Verschluß der Kammer kann natürlich auch auf verschiedene andere Weise erfolgen.

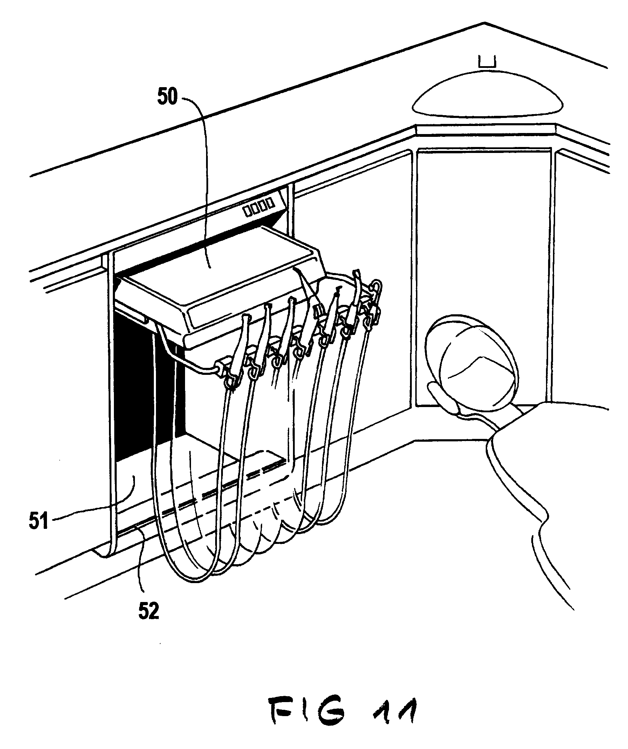

Wie aus Figur 10 hervorgeht, befindet sich in der Desinfektorkammer 41 ein sog. Peitschenabweiser in Form einer Stange 44, die dafür sorgt, daß die Peitschen nach Einschieben des Gerätekopfes in die Desinfektorkammer aufgerichtet, dabei die Instrumente 45 aus ihren Ablagen 46 (Fig. 9) abgehoben und in eine davor befindliche wannenartige Vertiefung 47 getaucht werden. Die Vertiefung 47 kann mit Reinigungsflüssigkeit gefüllt und gegebenenfalls mit einer Vorrichtung zur Abgabe von Ultraschall ausgestattet sein. In ihr kann auch eine spezielle Wascheinrichtung angeordnett sein, in der die Instrumente mit einem Wasser- oder Dampfstrahl behandelt werden.

Wie bei den zuvor beschriebenen Ausführungsbeispielen sind an verschiedenen Stellen des Gehäuses diverse Düsen 48 angeordnet, über die zur Außenreinigung der Teile Reinigungsflüssigkeit auf die Teile gestrahlt werden kann.

An dieser Stelle sei darauf hingewiesen, daß die soeben beschriebene Ausführungsform auch in Verbindung mit einer Konstruktion nach den Figuren 6 und 7 gesehen werden kann, d.h. auch bei jener Ausführung kann eine Möglichkeit vorgesehen werden, im abgelegten Zustand die Instrumente automatisch in eine entsprechend vorgesehene Wanne, in der sich Reinigungsflüssigkeit befindet, zu tauchen.

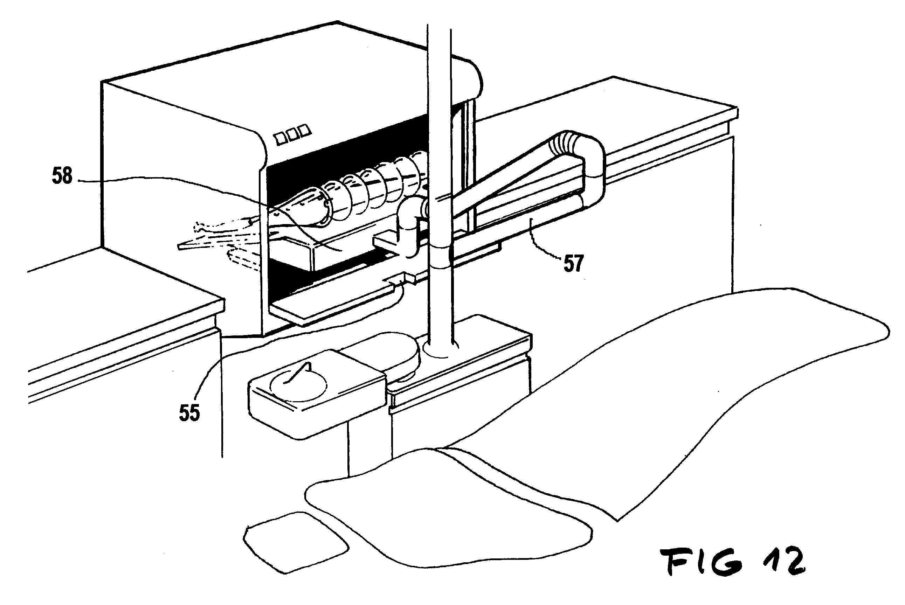

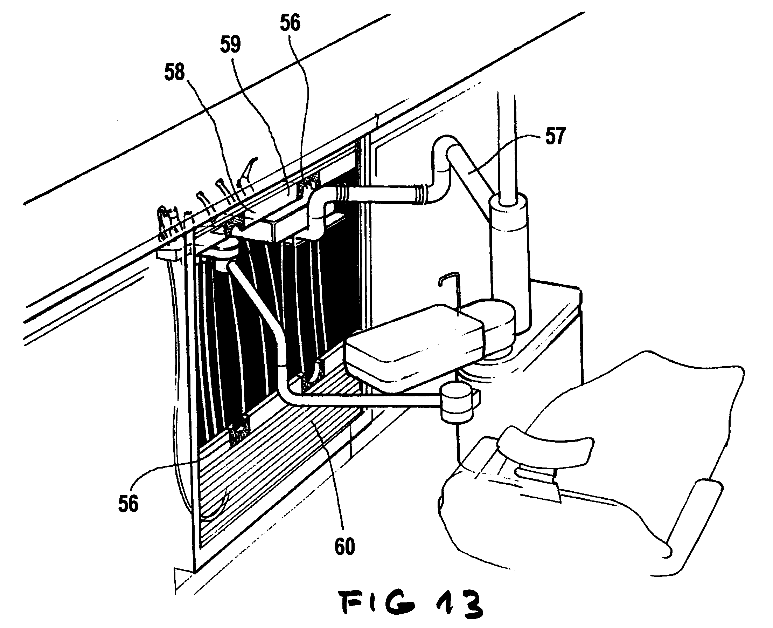

Die Figuren 11, 12 und 13 zeigen weitere Ausführungsformen einer Ausbildung der Desinfektorkammer. Während bei der Ausführungsform nach Figur 11 der gesamte Gerätekopf 50 in eine schrankartige Desinfektorkammer 51 eingefahren wird und diese dann mittels einer Jalousie 52 oder einer anderen Tür geschlossen wird, werden bei den Ausführungsformen nach den Figuren 12 und 13 lediglich die Geräteköpfe in die Desinfektorkammern eingefahren. Die Halterung bzw. die Tragarme, an denen die Geräteköpfe befestigt sind, ragen jeweils aus den Desinfektorkammern heraus. Entsprechend gestaltete Ausschnitte 55 und Dichtungen 56 in der Jalousie bzw. in mit dieser korrespondierenden Gehäuseteilen sorgen dafür, daß die beim Reinigen erforderliche Abdichtung gewährleistet ist.

## Patentansprüche

1. Verfahren zur Reinigung und Entkeimung eines zahnärtztlichen Gerätekopfes (1,22,35,50) der Ablagehalterungen (5,25,46) für schlauchgebundene Instrumente (6,26,46) umfaßt und ein Bedienfeld (8,28,48) enthält und der mit einer Handhabe (7) ausgestattet ist, durch die er in unterschiedliche Behandlungsstellungen positionierbar ist, **dadurch gekennzeichnet,** daß zumindest die Instrumente (6,26,45) mit den daran angeschlossenen Schläuchen (16,27), vorzugsweise auch die Handhabe (7) sowie das Bedienfeld (8,28,48) in betriebsfähigem Zustand, d.h. ohne vorherige Demontage der Teile, gereinigt und außen und innen entkeimt werden, indem sie in eine verschließbare Desinfektorkammer (15,29,41,51) gebracht werden, in der sie automatisch zunächst von außen durch Abstrahlen mit einem Reinigungsmedium oder in einem Ultraschallbad gereinigt werden, und in der anschließend die Außenflächen sowie die Zufuhrleitungen in den Schläuchen und die Kanäle in den Instrumenten innen mit einem Desinfektionsmedium entkeimt werden.

2. Verfahren nach Anspruch 1, **dadurch gekennzeichnet,** daß die Entkeimung der äußeren Flächen der Teile mit der Entkeimung der Medienkanäle und Medienleitungen in einem Prozeß erfolgt.

3. Verfahren nach Anspruch 1, **dadurch gekennzeichnet,** daß die Verfahrensschritte in einer extern des Gerätekopfes (35,50) angeordneten Desinfektorkammer (41,50) ablaufen.

4. Verfahren nach Anspruch 1 , **dadurch gekennzeichnet,** daß die Verfahrensschritte in einer im Gerätekopf (1,22) integrierten Desinfektorkammer (15,29) ablaufen.

5. Verfahren nach einem der Ansprüche 1 bis 4, **dadurch gekennzeichnet,** daß zum Reinigen ein unter Druck stehender Wasser-oder Dampfstrahl verwendet wird, der zumindest bei der Außenreinigung der Teile über Düsen (17,24,48) auf die Oberfläche der Teile mit hoher Energie aufgestrahlt wird.

6. Verfahren nach einem der Ansprüche 1 bis 4, **dadurch gekennzeichnet,** daß zum Entkeimen Dampf, Ozonwasser, Gas, Chemikalien oder Heißluft verwendet wird.

7. Verfahren nach einem der vorhergehenden Ansprüche, **dadurch gekennzeichnet,** daß besonders stark kontaminierte Teile, insbesondere die Instrumente, während des Verfahrensablaufs, vorzugsweise zwischen den Verfahrensschritten, in Reinigungsflüssigkeit getaucht werden.

8. Verfahren nach einem der vorhergehenden Ansprüche, **dadurch gekennzeichnet,** daß die Reinigung in einem Ultraschallbad erfolgt.

9. Verfahren nach einem der vorhergehenden Ansprüche, **dadurch gekennzeichnet,** daß die in der Desinfektorkammer vorhandene Feuchtigkeit durch Einblasen von warmer Luft und/oder durch Absaugen der Luft entfernt wird.

10. Einrichtung zur Durchführung des Verfahrens nach einem der Ansprüche 1 bis 9, **dadurch gekennzeichnet,** daß die Desinfektorkammer (15,29) integraler Bestandteil des Gerätekopfes (1,22) ist.

11. Einrichtung nach Anspruch 10 , **dadurch gekennzeichnet,** daß der Gerätekopf (1) als Instrumententisch ausgebildet ist, der bedienseitig eine Köcherleiste (4) mit Ablagehalterungen (5) für Instrumente (6) enthält, daß der Gerätekopf eine Desinfektorkammer (15) umfaßt , in die zumindest die Instrumente mit ihren Schläuchen (16) vor Ablauf des Verfahrens einbringbar sind, wobei die Einbringöffnung (21) durch ein Abdeckmittel (9) verschließbar ist, und daß in der Desinfektorkammer ein Düsensystem (17) zur Zufuhr des Reinigungsmediums angeordet ist.

12. Einrichtung nach Anspruch 11, **dadurch gekennzeichnet,** daß der Gerätekopf (1) mindestens einen in die Desinfektorkammer (15) einschwenkbaren Griff (7) enthält.

13. Einrichtung nach Anspruch 11, **dadurch gekennzeichnet,** daß der Gerätekopf (1) mit einem Bedienfeld (8) versehen ist.

14. Einrichtung nach einem der Ansprüche 11 bis 13, **dadurch gekennzeichnet,** daß als Abdeckmittel eine im eingebrachten Zustand der Teile die Einbringöffnung verschließende, sonst freigebende Jalousie(9) vorgesehen ist.

15. Einrichtung nach Anspruch 14, **dadurch gekennzeichnet,** daß als Abdeckmittel ein Band (9) verwendet ist, welches durch einen Seilzug (10) bewegt wird.

16. Einrichtung nach einem der Ansprüche 11 bis 15, **dadurch gekennzeichnet,** daß die Köcherleiste (4) mit Hilfe einer in Führungselementen (19) geführten Schlauchrückzugsvorrichtung(18)in der Desinfektorkammer (15) gehaltert ist.

17. Einrichtung nach Anspruch 10, **dadurch gekennzeichnet,** daß die Desinfektorkammer (29) durch Gehäuseteile (23) des Gerätekopfes (22) und einer diesen umgebenden Abdeckung (30) gebildet wird.

18. Einrichtung nach Anspruch 17, **dadurch gekennzeichnet,** daß der Gerätekopf (22) wannenartig ausgebildet ist und zumindest an seitlichen Randteilen (23a,23b) und am Boden (23c) eine Vielzahl von Düsen (24) zur Zufuhr des Reinigungsmediums angeordnet sind.

19. Einrichtung nach Anspruch 17, **dadurch gekennzeichnet,** daß der Gerätekopf (22,35) eine peitschenartige Instrumentenhalterung mit in der Nichtgebrauchsstellung schräg liegenden Instrumenten (26,45) und Schläuchen (27) aufweist, und in der Desinfektorkammer (29,41) ein Sperrmittel (44) angeordnet ist, welches bei Rückführung eines Instruments das Instrument in ein vor der Ablagehalterung befindliches Behältnis (47) eintauchen läßt.

20. Einrichtung nach einem der Ansprüche 10 bis 19, **dadurch gekennzeichnet,** daß der Träger für die Ablagehalterungen(25,46) der Instrumente (26,45) ein Bedienfeld (28) beinhaltet

21. Einrichtung zur Durchführung des Verfahrens nach einem der Ansprüche 1 bis 9, **dadurch gekennzeichnet,** daß die Desinfektorkammer (41,51) extern des Gerätekopfes (35,50) angeordnet ist.

22. Einrichtung nach Anspruch 22, **dadurch gekennzeichnet,** daß die Desinfektorkammer (41,51) integraler Bestandteil eines Schrankes (39) ist.

23. Einrichtung nach Anspruch 21, **dadurch gekennzeichnet,** daß die Desinfektorkammer (41,51) so ausgebildet ist, daß zumindest der Gerätekopf (35,50), vorzugsweise auch noch weitere Teile, insbesondere ein Ablagetablett (36) und/oder eine Speischale (37) darin untergebracht werden können.

24. Einrichtung nach Anspruch 23, **dadurch gekennzeichnet,** daß der Gerätekopf (50,58) von einem extern der Desinfektorkammer (41,51) befestigten mehrgliedrigen Tragarm (57) getragen ist, und die Abdeckung (42,52,59,60) gerätespezifische Aussparungen (55) und Dichtungen (56) für den Tragarm (57) enthält.
